# EUROPEAN PATENT SPECIFICATION

(11) **EP 0 349 505 B1**
(45) Date of publication and mention of the grant of the patent: **16.09.1998**
(21) Application number: 89850201.8
(22) Date of filing: 19.06.1989
(51) Int. Cl.: A61L 27/00, A61L 31/00

(54) **A novel surgical material**
Chirurgisches Material
Matériau chirurgical

(30) Priority: 27.06.1988 SE 8802414
(43) Date of publication of application: 03.01.1990
(60) Divisional application: 96202092.1
(73) Proprietor: Astra Aktiebolag, 151 85 Södertälje (SE)
(72) Inventor: Bowald, Staffan Folke, S-743 00 Storvreta (SE); Johansson, Eva Gunilla, S-412 61 Göteborg (SE)
(74) Representative: As Sivborg, Susanne Birgitta

(56) References cited:
- WO-A-86/02843
- FR-A- 2 060 524
- US-A- 4 355 426
- US-A- 4 603 070
- US-A- 4 603 695
- US-A- 4 719 246
- P. A. Holmes, Phys. Technol., Vol. 16, p. 32 - 36 (1985).
- N. D. Miller & D. F. Williams, Biomaterials, Vol. 8, p. 129 - 137 (1987).
- D. F. Williams & N. D. Miller in: Biomaterials and clinical applications, p. 471 - 476, Elsevier, Amsterdam, 1987.
- M. Vert, S. M. Li, G. Spenlehauer & P. Guerin, J. Mat. Sci.: Materials in Medicine, Vol. 3, p. 432 - 446 (1992).

## Description

### Technical Field

The present invention is related to a material for separation of injured tissue at healing processes. It is further related to a process for preparation of such material, and to use of the material in healing processes.

### Background of the Invention

In healing of injuries in soft tissue, scar tissue develops, which in many cases disturbs the function of the damaged organ and adjacent organs. This problem primarily occurs in healing of internal organs, while the problems in healing of skin and external mucosae may also be of cosmetic nature. Reference to injury and injured soft tissue herein primarily relates to incisions and other injuries caused by surgical operations in the organ subject to surgical correction, as well as in covering and adjacent organs. Among injuries caused by surgical operations is included injuries caused by surgical correction of congenital defects e.g. fistulas. The material according to the invention may also advantageously be employed in healing of injuries caused by external violence e.g. in accidents. A purpose with the invention is to facilitate and improve the healing by locking out undesired cells, other tissue and/or foreign particles. For this purpose, porous cloth of polytetrafluoroethylene such as Gore-Tex^{R} is currently in use. The disadvantage with this is that foreign material will remain in the body, which may cause problems. Bowald et al. in The Lancet No. 8056, January 21, 1978, page 153 describes the use of a knitted mesh of polyglactin 910 (Vicryl^{R}) as an arterial substitute. A preclotted mesh was sutured as a patch graft or as an end to en tube in the thoratic aorta in pigs. This coarse-mesh material is only useful by deposition of fibrin intermingled with platelets and red blood cells in the mesh spaces. Further studies on coarse-mesh polyglactin material were reported in Surgery vol. 86, no 5, pp. 722-729, 1979, in Scand J Thor Cardiovasc Surg 15:91-94, 1981, in Muscle & Nerve 5:54-57, 1982 and in Acta Chir Scand 146:391-395, 1980. SE 8604571-3 describes the use of resorbable and non resorbable membranes for accelerating bone formation and bone healing. However, cellular processes of resorptive type are indicated as undesirable, as they may delay bone formation and damage the newly formed bone.

US-A-4603695 (Ikada et al./Japan Medical Supply Co., Ltd.) discloses a number of materials which may be used to prevent adhesion of vital tissues which are absorbable by degradation in vital tissues. Polymers and copolymers of lactic acid are preferred, though a polyester of β-hydroxybutylcarboxylic acid is also mentioned.

Biomaterials, vol. 8, 129-137, 1987 (Miller et al.) discloses a study on the biodegradation of poly-β-hydroxybutyrate (PHB) homopolymer and poly-β-hydroxybutyratehydroxyvalerate copolymers. The conclusion drawn by the authors is that PHB is generally not biodegradable in vivo. Furthermore, they state that the biodegradation of implanted PHB and its copolymers cannot be predicted with confidence.

### Description of the Invention

According to the invention it has now been made possible to avoid such problems as mentioned above in healing of soft tissue. The invention provides the use of polyhydroxybutyric acid polymers or copolymers of hydroxybutyric acid and hydroxyvaleric acid for preparing a porous, bioresorbable flexible sheet for tissue separation and stimulation of tissue regeneration at healing processes in injured soft tissue in mammals including man, said sheet having a pore size permitting passage of water and salts through the sheet but which locks out cells and other tissue particles, said sheet being adapted to replace a part of the pericardium. Using this material has been found to cause a specific stimulating effect on formation of macrophages in soft tissue. The macrophages release a growth factor which stimulates tissue healing. Using this material of the invention does not require preclotting or the presence of blood for functioning.

According to a preferred embodiment of the invention the material has a pore size up to 30 µm, preferentially 0.1-10 µm. The sheet thickness may be between 1 µm and 5 mm, but is preferably 10 µm to about 1 mm.

The invention also provides a porous, bioresorbable flexible sheet for tissue separation and stimulation of tissue regeneration at healing processes in injured soft tissue in mammals including man, said sheet being made of polyhydroxybutyric acid polymers or copolymers of hydroxybutyric acid and hydroxyvaleric acid and having a pore size permitting passage of water and salts through the sheet but which locks out cells and other tissue particles, said sheet being adapted to replace a part of the pericardium.

The material may according to the invention be prepared according to the following processes, of which non-woven technique, precipitation and laser technique are preferred:

### Non-woven

Non-woven fibrous material is prepared as described in U.S. Patent No. 4 603 070. Fibres are produced from a melt or solution of the polymer by pressing the material through a perforated outlet. The fibres are spread randomly, or with a main orientation, on a support (a still glass plate - a mobile net ribbon - other mould). In this manner a porous "cloth" is obtained which may be given varying porosity by modification of fibre dimension, spreading method, material thickness and/or by working up with heat/compression. The thickness of the cloth is preferably 300-500 µm.

### Perforation

A homogenous film/cloth of the material may be perforated by e.g. laser technique to achieve porosity. In particular, a weak so called excimer laser may be employed together with a template of perforated stainless steel.

### Precipitation (not applicable to PGA)

The polymer is dissolved in a solvent which may be selected from a first group comprising dimethyl formamide (DMF), dimethylacetamide (DMA), dimethyl sulphoxide (DMSO), and tetrahydrofuran (THF), or from a second group comprising chlorinated hydrocarbons such as chloroform and methylene chloride. Precipitation of the polymer may be achieved with a precipitation agent, which with the first group of solvents suitably is water, possibly with an addition of up to 20 % solvent. With the second group of solvents ethanol and other lower alcohols may be used as a precipitation agent, possibly with an addition of up to 20 % solvent. Both the solvent and the precipitation agent may be a mixture. Temperature and time at the precipitation may be selected to achieve any desired pore size.

### Effervescence

By admixture of an effervescing agent which releases gas e.g. in contact with water (at precipitation) or on heating (in a melt).

### Leaching

Soluble particles, for example salt, are suspended in a solution of the polymer /admixed into a melt thereof. After evaporation/solidification the particles are washed out of the material by leaching in a suitable solvent for the particles (but not for the polymer). This washing can be done completely, or partially provided that a non-toxic salt such as NaCl is employed, whereby the residual amount of salt may be allowed to leach out after implanting the material into the body.

The material is used according to the invention in healing of soft tissue, i.e. tissue that does not consist of cartilage, bone or teeth. Specifically the material is for replacing part of the pericardium.

Suitable bioresorbable materials for the purposes of the present invention may readily be chosen by one skilled in the art, e.g. among those that are either commercially available or have been described in literature or will be available in the future. As examples of such bioresorbable materials may be mentioned polymers based on polyglycolic acid (PGA), copolymers of glycolic acid and lactic acid, copolymers of lactic acid and ε-aminocapronic acid, and various lactide polymers. PGA esters are, e.g. described in U.S. Patent No. 3 463 658, while copolymers of glycolic acid and lactic acid are described e.g. in U.S. Patent No. 3 982 543. Homo and copolymers of lactic acid are described in e.g. U.S. Patent No. 3 636 956. Examples of commercially available materials are Vicryl^{R} (a copolymer of 90 % glycolic acid sold by Ethicon, Sommerville, N.Y., U.S.A. - also known as Polyglactin) and Dexon^{R} (Davies & Geck, Pearl River, N.Y., U.S.A.). Further examples are polydesoxazon (PDS) (Ethicon, U.S.A.), polyhydroxybutyric acid (PHB), copolymers of hydroxybutyric acid and hydroxyvaleric acid (PHBV), polyesters of succinic acid, and crosslinked hyaluronic acid. As suggested above, mixtures of the above-mentioned materials may equally well be employed. One skilled in the art would have no difficulty to modify such bioresorbable materials depending on current needs, e.g. with regard to resorption time, strength etc.

Possibly, growth factors may be included in the porous structure, either deposited in the pores or included in the bioresorbable material for slow release of growth factor.

The sheet-formed material according to the invention may suitably, in particular in application for strong muscles and in other locations where the material is subject to strong load, be combined with a resorbable armament e.g. a woven or knitted cloth.

The invention is further described with reference to the following examples.

### Example 1

Preparation of a sheet material for replacement of a part of the pericardium.

5 ml of a solution of 10 g Biopol (PHBV, 20 % hydroxyvaleric acid) in 100 ml dimethylacetamide (about 50 °C) was spread on a glass plate. The glass plate was thereafter placed in water of ambient temperature for 12 hours. In this manner a porous patch (8 x 8 cm) was formed having about 1 mm thickness. The patch was washed in water, dried, packed and sterilized (ethylene oxide).

### Example 2

Use for healing of pericardiac defects.

In connection with cardiac surgery, difficulties occur almost always in closing the pericardium. This results in the pericardium often being left open. The result is adhesion which causes severe difficulties on re-operations and also an decreased motility of the heart, the function of which is impaired.

In connection with cardiac operations on sheep the defect caused was replaced with a patch of tissue-compatible resorbable polymer prepared according to Example 1. The patch was stitched into the defect by a continuous suture. When the animal after four months of healing was sacrificed and autopsy was performed, virtually normal pericardiac tissue was found to be formed without growing together with the heart surface, and the heart had been freely motile in the pericardium.

### Example 3

Producing a nonwoven patch for reconstruction of pericardium.

The nonwoven material was made from solution spun PHB-fibres pressed together to a patch (produced in accordance with US patent 4,603,070). Patch thickness was about 0.4 mm with about 70 per cent pore volume, patch size 15 x 15 cm. The patch was sterilized in ethylene oxide.

### Example 4

Nonwoven PHB patches, produced according to Example 3, was used to replace a part of the pericardium in 10 sheep. The animals have been followed up for more than one year after the operation and have been sacrificed at different times. After two months regeneration of the pericardium had started, a very loose adhesion could be found. In the tissue a very active phagocytosis, with macrophages as the dominating type of cells, could be seen. No other kind of inflammation was present.

Later there were no signs of adhesion and already after four months a healing, very much like normal pericardium could be seen. The inner side was very smooth and glossy and mesothelial cells were present, which means that real pericardium had regenerated.

Up to ten months a slight darkness of the patch area could be observed due to partly remaining polymer. The darkness disappeared when all polymer was resorbed.

## Claims

1. Use of polyhydroxybutyric acid polymers or copolymers of hydroxybutyric acid and hydroxyvaleric acid for preparing a porous, bioresorbable flexible sheet for tissue separation and stimulation of tissue regeneration at healing processes in injured soft tissue in mammals including man, said sheet having a pore size permitting passage of water and salts through the sheet but which locks out cells and other tissue particles, said sheet being adapted to replace a part of the pericardium.

2. Use in accordance with claim 1, in which the pore size is up to 30 µm, preferentially 0.1-10 µm.

3. Use in accordance with claim 1 or 2, in which the thickness of the sheet is about 10 µm-1 mm.

4. Use accordance with any one of the preceding claims, said sheet being combined with a resorbable reinforcement.

5. A porous, bioresorbable flexible sheet for tissue separation and stimulation of tissue regeneration at healing processes in injured soft tissue in mammals including man, said sheet being made of polyhydroxybutyric acid polymers or copolymers of hydroxybutyric acid and hydroxyvaleric acid and having a pore size permitting passage of water and salts through the sheet but which locks out cells and other tissue particles, said sheet being adapted to replace a part of the pericardium.

6. A sheet in accordance with claim 5, in which the pore size is up to 30 µm, preferentially 0.1-10 µm.

7. A sheet in accordance with claim 6, in which the thickness of the sheet is about 10 µm-1 mm.

8. A sheet in accordance with any one of claims 5 to 7, said sheet being combined with a resorbable reinforcement.

9. A process for preparing a porous, bioresorbable flexible sheet according to any one of claims 5 to 8, characterized by the step of forming the sheet by a non-woven technique.

10. A process for preparing a porous, bioresorbable flexible sheet according to any one of claims 5 to 8, characterized by the step of forming the sheet by precipitation of the polymer or copolymer.

11. A process for preparing a porous, bioresorbable flexible sheet according to any one of claims 5 to 8, characterized by the steps of forming the sheet and then perforating said sheet by a laser technique.

## Patentansprüche

1. Verwendung von Polyhydroxybuttersäurepolymeren oder Copolymeren aus Hydroxybuttersäure und Hydroxyvaleriansäure zur Herstellung eines porösen, bioresorbierbaren flexiblen Flächengebildes zur Gewebetrennung und Stimulierung der Geweberegeneration bei Heilprozessen in verletztem Weichgewebe von Säugern einschließlich Menschen, wobei das Flächengebilde eine Porengröße aufweist, die den Durchgang von Wasser und Salzen durch das Flächengebilde gestattet, aber Zellen und andere Gewebeteilchen aussperrt, und als Ersatz eines Teils des Pericardiums ausgeführt ist.

2. Verwendung nach Anspruch 1, wobei die Porengröße bis zu 30 µm und vorzugsweise 0,1-10 µm beträgt.

3. Verwendung nach Anspruch 1 oder 2, wobei das Flächengebilde etwa 10 µm - 1 mm dick ist.

4. Verwendung nach einem der vorhergehenden Ansprüche, wobei das Flächengebilde mit einer resorbierbaren Verstärkung kombiniert wird.

5. Poröses, bioresorbierbares flexibles Flächengebilde zur Gewebetrennung und Stimulierung der Geweberegeneration bei Heilprozessen in verletztem Weichgewebe von Säugern einschließlich Menschen, wobei das Flächengebilde aus Polyhydroxybuttersäurepolymeren oder Copolymeren aus Hydroxybuttersäure und Hydroxyvaleriansäure aufgebaut ist, eine Porengröße aufweist, die den Durchgang von Wasser und Salzen durch das Flächengebilde gestattet, aber Zellen und andere Gewebeteilchen aussperrt, und als Ersatz eines Teils des Pericardiums ausgeführt ist.

6. Flächengebilde nach Anspruch 5, wobei die Porengröße bis zu 30 µm und vorzugsweise 0,1-10 µm beträgt.

7. Flächengebilde nach Anspruch 6, das 10 µm - 1 mm dick ist.

8. Flächengebilde nach einem der Ansprüche 5 bis 7, das mit einer resorbierbaren Verstärkung kombiniert ist.

9. Verfahren zur Herstellung eines porösen, bioresorbierbaren Flächengebildes nach einem der Ansprüche 5 bis 8, dadurch gekennzeichnet, daß man das Flächengebilde nach einer Vliesmethode herstellt.

10. Verfahren zur Herstellung eines porösen, bioresorbierbaren Flächengebildes nach einem der Ansprüche 5 bis 8, dadurch gekennzeichnet, daß man das Flächengebilde durch Fällung des Polymers oder Copolymers herstellt.

11. Verfahren zur Herstellung eines porösen, bioresorbierbaren Flächengebildes nach einem der Ansprüche 5 bis 8, dadurch gekennzeichnet, daß man das Flächengebilde herstellt und dann nach einer Lasermethode perforiert.

## Revendications

1. Utilisation de polymères du poly(acide hydroxybutyrique) ou de copolymères de l'acide hydroxybutyrique et de l'acide hydroxyvalérique pour la préparation d'une feuille flexible poreuse et biorésorbable pour la séparation de tissu et la stimulation de la régénération tissulaire lors des processus de cicatrisation de tissu mou blessé chez les mammifères, y compris l'homme, ladite feuille présentant une taille de pore permettant le passage d'eau et de sels au travers de la feuille mais arrêtant les cellules et autres particules tissulaires, ladite feuille étant adaptée pour remplacer une partie du péricarde.

2. Utilisation suivant la revendication 1, dans laquelle la taille de pore va jusqu'à 30 µm, de préférence de 0,1 à 10 µm.

3. Utilisation suivant la revendication 1 ou 2, dans laquelle l'épaisseur de la feuille est d'environ 10 µm à 1 mm.

4. Utilisation suivant l'une quelconque des revendications précédentes, ladite feuille étant combinée à un renforcement résorbable.

5. Feuille flexible poreuse et biorésorbable pour la séparation de tissu et la stimulation de la régénération tissulaire lors des processus de cicatrisation de tissu mou blessé chez les mammifères, y compris l'homme, ladite feuille étant faite de polymères du poly(acide hydroxybutyrique) ou de copolymères de l'acide hydroxybutyrique et de l'acide hydroxyvalérique et présentant une taille de pore permettant le passage d'eau et de sels au travers de la feuille mais arrêtant les cellules et autres particules tissulaires, ladite feuille étant adaptée pour remplacer une partie du péricarde.

6. Feuille suivant la revendication 5, dans laquelle la taille des pores va jusqu'à 30 µm, de préférence de 0,1 à 10 µm.

7. Feuille suivant la revendication 6, dans laquelle l'épaisseur de la feuille est d'environ 10 µm à 1 mm.

8. Feuille suivant l'une quelconque des revendications 5 à 7, ladite feuille étant combinée à un renforcement résorbable.

9. Procédé de préparation d'une feuille flexible poreuse et biorésorbable suivant l'une quelconque des revendications 5 à 8, caractérisé par l'étape de formation de la feuille par une technique de non tissé.

10. Procédé de préparation d'une feuille flexible poreuse et biorésorbable suivant l'une quelconque des revendications 5 à 8, caractérisé par l'étape de formation de la feuille par précipitation du polymère ou du copolymère.

11. Procédé de préparation d'une feuille flexible poreuse et biorésorbable suivant l'une quelconque des revendications 5 à 8, caractérisé par les étapes de formation de la feuille puis de perforation de ladite feuille par une technique au laser.
